# EUROPEAN PATENT SPECIFICATION

(11) **EP 2 185 086 B1**
(45) Date of publication and mention of the grant of the patent: **21.08.2019**
(21) Application number: 08794704.0
(22) Date of filing: 24.07.2008
(51) Int. Cl.: A61B 17/58, A61F 2/44, A61B 17/70

(54) **INTERSPINOUS SPACER**
INTERSPINÖSES ABSTANDSGLIED
ESPACEUR INTERÉPINEUX

(30) Priority: 24.07.2007 US 961741 P
(43) Date of publication of application: 19.05.2010
(73) Proprietor: Vertiflex, Inc., San Clemente, CA 92673 (US)
(72) Inventor: ALTARAC, Moti, Irvine, CA 92603 (US); TEBBE, Shawn, Dromiskin Co. Louth (IE); REGLOS, Joey, Camia, Lake Forest, CA 92630 (US); CHENG, Yang, Foothill Ranch, CA 92610 (US); KADABA, Murali, P., Foster City, CA 94404 (US); KIM, Daniel, H., Houston, CA 77007 (US)
(74) Representative: Donald, Jenny Susan
(86) International application number: PCT/US2008/008983
(87) International publication number: WO 2009/014728

(56) References cited:
- WO-A1-2006/064356
- WO-A2-2007/075788
- US-A1- 2005 245 937
- US-A1- 2006 271 194
- US-A1- 2006 276 801
- US-A1- 2007 032 790
- US-B2- 6 645 207
- US-B2- 7 179 225

## Description

### FIELD

The present invention generally relates to medical devices, in particular, implants for placement between adjacent spinous processes of a patient's spine.

### BACKGROUND

With spinal stenosis, the spinal canal narrows and pinches the spinal cord and nerves, causing pain in the back and legs. Typically, with age, a person's ligaments may thicken, intervertebral discs may deteriorate and facet joints may break down-all contributing to the condition of the spine characterized by a narrowing of the spinal canal. Injury, heredity, arthritis, changes in blood flow and other causes may also contribute to spinal stenosis.

Doctors have been at the forefront with various treatments of the spine including medications, surgical techniques and implantable devices that alleviate and substantially reduce debilitating pain associated with the back. In one surgical technique, a spacer is implanted between adjacent interspinous processes of a patient's spine. The implanted spacer opens the spinal canal, maintains the desired distance between vertebral body segments, and as a result, avoids impingement of nerves and relieves pain. For suitable candidates, an implantable interspinous spacer may provide significant benefits in terms of pain relief.

Any surgery is an ordeal. However, the type of device and how it is implanted has an impact. For example, one consideration when performing surgery to implant an interspinous spacer is the size of the incision that is required to allow introduction of the device. Small incisions and minimally invasive techniques are generally preferred as they affect less tissue and result in speedier recovery times. WO2007075788 discloses devices include an expandable spacer having an undeployed configuration and a deployed configuration, wherein the spacer has axial and radial dimensions for positioning between the spinous processes of adjacent vertebrae. As such, there is a need for interspinous spacers that work well with surgical techniques that are minimally invasive for the patient. The present invention sets forth such a spacer.

### SUMMARY

According to one aspect of the invention, an implantable spacer for placement between adjacent spinous processes is provided. The spacer includes a body defining a longitudinal axis. A first arm and a second arm are connected to the body and capable of movement with respect to the body. Each arm defines a configuration for receiving a spinous process and has a proximal caming surface. The spacer further includes an actuator assembly connected to the body. The actuator assembly includes an actuator having at least one bearing surface, a shaft connected to the actuator and configured for movement with respect to the body; and a spindle. The actuator assembly is configured to move relative to the body such that rotation of the spindle moves the actuator such that the at least one bearing surface contacts at least one of the caming surfaces to move both of the arms from an undeployed configuration to a deployed configuration in which the arms receive adjacent spinous processes.

According to another aspect of the invention, an implantable spacer for placement between adjacent spinous processes is disclosed. The implant includes a body defining a longitudinal axis. A first arm and a second arm are both connected to the body and capable of movement with respect to the body. Each arm has a configuration for receiving a spinous process and each arm has a proximal caming surface. The spacer further includes an actuator connected to the body and configured to move relative to the body to deploy the arms from an undeployed configuration. In the deployed configuration, the arms seat adjacent spinous processes. The spacer also includes a lock configured to provide resistance to keep the arms in place.

According to another aspect of the invention, a spinal implant for relieving pain and implantable between a superior spinous process and an inferior spinous process is disclosed. The implant includes a body connected prior to implantation to at least one arm. The at least one arm is movable with respect to the body into at least one configuration that is adapted to laterally stabilize and secure the implant with respect to an adjacent spinous process. In one variation, the implant includes a first arm for laterally stabilizing the body with respect to the superior spinous process and a second arm for laterally stabilizing the body with respect to the inferior spinous process.

According to another aspect of the invention, a spinal implant for relieving pain and implantable between a superior spinous process and an inferior spinous process is disclosed. The implant includes a body connected prior to implantation to at least one arm. The at least one arm is movable with respect to the body into at least one configuration that is adapted to laterally stabilize and secure the body with respect to an adjacent spinous process. In one variation, the implant includes a first arm for laterally stabilizing the body with respect to the superior spinous process and a second arm for laterally stabilizing the body with respect to the inferior spinous process. The implant includes a collapsed configuration in which a first end of the first arm and a first end of the second arm form the leading edge of the implant.

According to another aspect of the invention, a spinal implant for relieving pain and implantable between a superior spinous process and an inferior spinous process is disclosed. The implant includes a body connected prior to implantation to at least one arm. The at least one arm is movable with respect to the body into at least one configuration that is adapted to laterally stabilize and secure the body with respect to an adjacent spinous process. In one variation, the implant includes a first arm for laterally stabilizing the body with respect to the superior spinous process and a second arm for laterally stabilizing the body with respect to the inferior spinous process. A second end of the first arm is hinged to the distal end of the body and a second end of the second arm is hinged to the distal end of the body. In one variation, the first and second arms are configured to rotate approximately 90 degrees about their hinged ends into a deployed configuration. In one variation, wherein when rotated approximately 90 degrees, the first and second arms are in a configuration that is adapted to laterally stabilize/secure the body with respect to adjacent spinous processes. In another variation, wherein after rotation of approximately 90 degrees, each of the first and second arms are configured to translate away from the body such that the arms are closer to their respective spinous processes.

### BRIEF DESCRIPTION OF THE DRAWINGS

The invention is best understood from the following detailed description when read in conjunction with the accompanying drawings. It is emphasized that, according to common practice, the various features of the drawings are not to-scale. On the contrary, the dimensions of the various features are arbitrarily expanded or reduced for clarity.
FIG. 1a illustrates a perspective view of a spacer according to the present invention.
FIG. 1b illustrates a side view of a spacer according to the present invention.
FIG. 1c illustrates a top view of a spacer according to the present invention.
FIG. 1d illustrates a cross-sectional view of the spacer of FIG. 1c taken along line A-A according to the present invention.
FIG. 1e illustrates an end view of a spacer according to the present invention.
FIG. If illustrates an exploded perspective view of a spacer according to the present invention.
FIG. 2a illustrates a perspective view of half of a body of a spacer according to the present invention.
FIG. 2b illustrates a side view of half of a body of a spacer according to the present invention.
FIG. 3a illustrates a perspective view of a superior arm of a spacer according to the present invention.
FIG. 3b illustrates a back view of a superior arm of a spacer according to the present invention.
FIG. 3c illustrates a side view of a superior arm of a spacer according to the present invention.
FIG. 3d illustrates a perspective view of an inferior arm of a spacer according to the present invention.
FIG. 3e illustrates a back view of an inferior arm of a spacer according to the present invention.
FIG. 3f illustrates a side view of an inferior arm of a spacer according to the present invention.
FIG. 4a illustrates a perspective view of a spindle of an actuator assembly of a spacer according to the present invention.
FIG. 4b illustrates a top view of a spindle of an actuator assembly of a spacer according to the present invention.
FIG. 4c illustrates a cross-sectional view of the spindle of FIG. 4b taken along line F-F according to the present invention.
FIG. 4d illustrates a perspective view of a lock according to the present invention.
FIG. 4e illustrates a top view of a lock according to the present invention.
FIG. 4f illustrates a partial cross-sectional top view of a lock, body and spindle according to the present invention.
FIG. 5a illustrates a side view of a spacer in a closed, undeployed configuration according to the present invention.
FIG. 5b illustrates a side view of a spacer in a partially deployed configuration according to the present invention.
FIG. 5c illustrates a side view of a spacer in a deployed configuration according to the present invention.
FIG. 6a illustrates a side, cross-sectional view of a spacer in a closed, undeployed configuration according to the present invention.
FIG. 6b illustrates a side, cross-sectional view of a spacer in a partially deployed configuration according to the present invention.
FIG. 6c illustrates a side, cross-sectional view of a spacer in a deployed configuration according to the present invention.
FIG. 7a illustrates a side, semi-transparent view of a spacer in a closed undeployed configuration according to the present invention.
FIG. 7b illustrates a side, semi-transparent view of a spacer in a partially deployed configuration according to the present invention.
FIG. 7c illustrates a side, semi-transparent view of a spacer in a deployed configuration according to the present invention.
FIG. 8 illustrates a side view of half of a body of a spacer according to the present invention.
FIG. 9a illustrates a perspective view of a superior arm of a spacer according to the present invention.
FIG. 9b illustrates a perspective view of an inferior arm of a spacer according to the present invention.
FIG. 10a illustrates a perspective view of half of a body of a spacer according to the present invention.
FIG. 10b illustrates a side view of half of a body of a spacer according to the present invention.
FIG. 11a illustrates a perspective view of a superior arm of a spacer according to the present invention.
FIG. 11b illustrates a back view of a superior arm of a spacer according to the present invention.
FIG. 11c illustrates a side view of a superior arm of a spacer according to the present invention.
FIG. 11d illustrates a perspective view of an inferior arm of a spacer according to the present invention.
FIG. 11e illustrates a back view of an inferior arm of a spacer according to the present invention.
FIG. 11f illustrates a side view of an inferior arm of a spacer according to the present invention.
FIG. 12a illustrates a side, semi-transparent view of a spacer in a closed, undeployed configuration according to the present invention.
FIG. 12b illustrates a side, semi-transparent view of a spacer in a partially deployed configuration according to the present invention.
FIG. 12c illustrates a side, semi-transparent view of a spacer in a deployed configuration according to the present invention.
FIG. 12d illustrates a side, semi-transparent view of a spacer in a deployed and extended configuration according to the present invention.
FIG. 13a illustrates a perspective view of half of a body of a spacer according to the present invention.
FIG. 13b illustrates a side view of half of a body of a spacer according to the present invention.
FIG. 14a illustrates a perspective view of a superior arm of a spacer according to the present invention.
FIG. 14b illustrates a back view of a superior arm of a spacer according to the present invention.
FIG. 14c illustrates a side view of a superior arm of a spacer according to the present invention.
FIG. 14d illustrates a perspective view of an inferior arm of a spacer according to the present invention.
FIG. 14e illustrates a back view of an inferior arm of a spacer according to the present invention.
FIG. 14f illustrates a side view of an inferior arm of a spacer according to the present invention.
FIG. 15a illustrates a side view of a spacer in a closed, undeployed configuration according to the present invention.
FIG. 15b illustrates a side view of a spacer in a partially deployed configuration according to the present invention.
FIG. 15c illustrates a side view of a spacer in a deployed configuration according to the present invention.
FIG. 15d illustrates a side view of a spacer in a deployed and extended configuration according to the present invention.
FIG. 16a illustrates a side, cross-sectional view of a spacer in a closed, undeployed configuration according to the present invention.
FIG. 16b illustrates a side, cross-sectional view of a spacer in a partially deployed configuration according to the present invention.
FIG. 16c illustrates a side, cross-sectional view of a spacer in a deployed configuration according to the present invention.
FIG. 16d illustrates a side, cross-sectional view of a spacer in a deployed and extended configuration according to the present invention.
FIG. 17a illustrates a side, semi-transparent view of a spacer in a closed undeployed configuration according to the present invention.
FIG. 17b illustrates a side, semi-transparent view of a spacer in a partially deployed configuration according to the present invention.
FIG. 17c illustrates a side, semi-transparent view of a spacer in a deployed configuration according to the present invention.
FIG. 17d illustrates a side, semi-transparent view of a spacer in a deployed and extended configuration according to the present invention.
FIG. 18a illustrates a side view of an insertion instrument connected to a spacer in a closed, undeployed configuration according to the present invention.
FIG. 18b illustrates a side view of an insertion instrument connected to a spacer in a partially deployed configuration according to the present invention.
FIG. 18c illustrates a side view of an insertion instrument connected to a spacer in a deployed configuration according to the present invention.
FIG. 18d illustrates a side view of an insertion instrument connected to a spacer in a deployed and extended configuration according to the present invention.
FIG. 19 illustrates a spacer according to the present invention deployed in an interspinous process space between two vertebral bodies and a supraspinous ligament.

### DETAILED DESCRIPTION

Before the subject devices, systems and methods are described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs.

It must be noted that as used herein and in the appended claims, the singular forms "a", "an", and "the" include plural referents unless the context clearly dictates otherwise. Thus, for example, reference to "a spinal segment" may include a plurality of such spinal segments and reference to "the screw" includes reference to one or more screws and equivalents thereof known to those skilled in the art, and so forth.

All publications mentioned herein are incorporated herein by reference to disclose and describe the methods and/or materials in connection with which the publications are cited. The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

The present invention is described in the accompanying figures and text as understood by a person having ordinary skill in the field of spinal implants and implant delivery instrumentation.

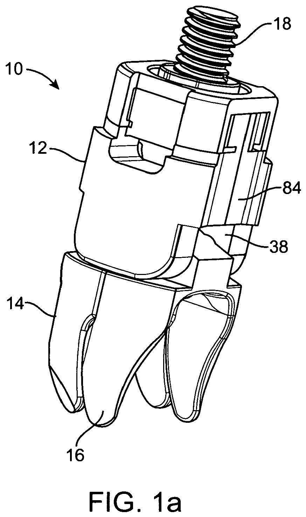

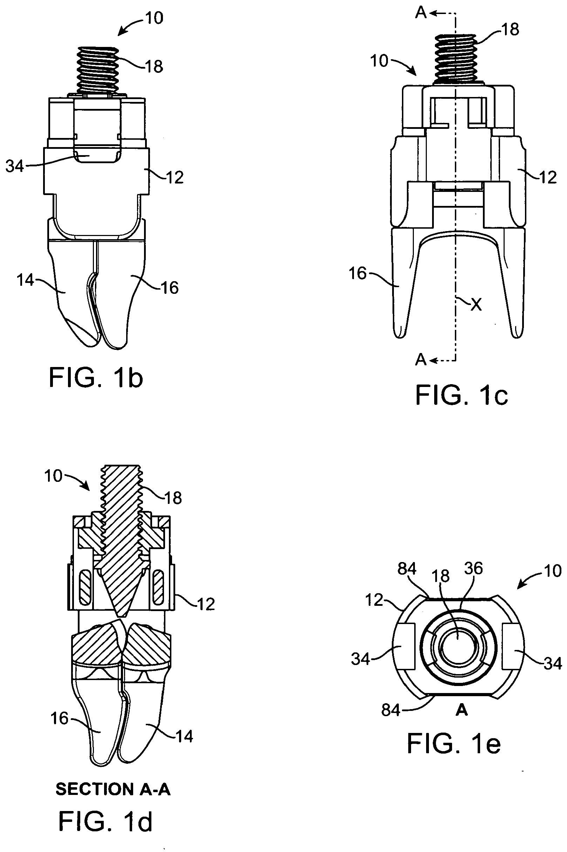

With reference to FIGs. 1a-1f, various views of a spacer 10 according to the present invention are shown. The spacer 10 includes a body 12 connected to a superior extension member or arm 14, an inferior extension member or arm 16, and an actuator assembly 18.

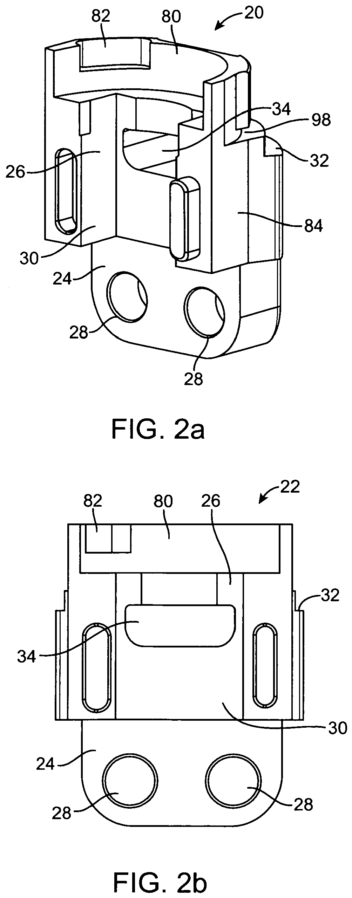

Turning now to FIGs. 2a-2b, the body 12 will now be described. The body 12 is shown to have a clamshell construction with a left body piece 20 (shown in FIGs. 2a) joined to a right body piece 22 (shown in FIGs. 2b) to capture arms 14, 16 inside. With the right and left body pieces 20, 22 joined together, the body 12 is generally cylindrical. The spacer body 12 has a cross-sectional size and shape that allows for implantation between adjacent spinous processes and facilitates delivery into a patient through a narrow port or cannula.

The inside of the body 12 defines an arm receiving portion 24 and an actuator assembly receiving portion 26 with features formed in each of the left and right body pieces 20, 22 that together define the arm and actuator assembly receiving portions 24, 26. In one variation, the arm receiving portion 24 includes slots or openings 28 that receive pins formed on the arms 14, 16 such that the pins rotate and/or translate inside the openings 28. The actuator assembly receiving portion 26 includes a passageway 30. The actuator assembly receiving portion 26 includes a spindle receiving portion 80 formed by the two joined pieces 20, 22 to form a ledge. The actuator assembly receiving portion 26 also includes at least one lock receiving portion 82. Other features include a tongue and groove for mating with the opposite clamshell.

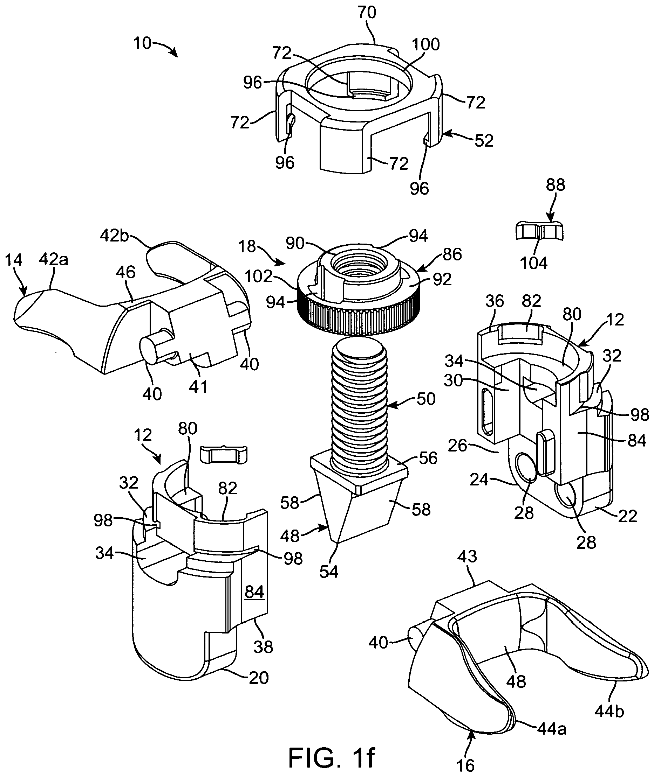

The outside of the body 12 defines a ledge 32 along at least a portion of the periphery and at least one or continous undercut 98. Notches 34 are formed at opposite locations as also shown in FIG. 1b. The notches 34 are configured for pronged attachment to a spacer delivery instrument. When joined together, the left and right body pieces 20, 22 define a proximal opening 36 (as seen in FIG. 1e) and a distal opening 38 (as seen in FIG. 1a) in the body 12. A longitudinal scallop 84 (also shown in FIGs. 1a, 1e, 1f and 2a) extending from the proximal end of the spacer to the distal end is formed in the outer surface of the body 12 to facilitate placement of the spacer 10 between and to conform to the anatomy of adjacent interspinous processes. On one variation, two oppositely located londigutinal scallops 84 are formed in the outer surface of the body 12 such that one scallop 84 faces the superior spinous process and the other scallop 84 faces the inferior spinous process. In one variation, the distance between oppositely located longitudinal scallops 84 (as best seen in FIG. 1e) is approximately 8.0 millimeters imparting the spacer 10 with a low profile advantageous for insertion between closely spaced or "kissing" spinous processes.

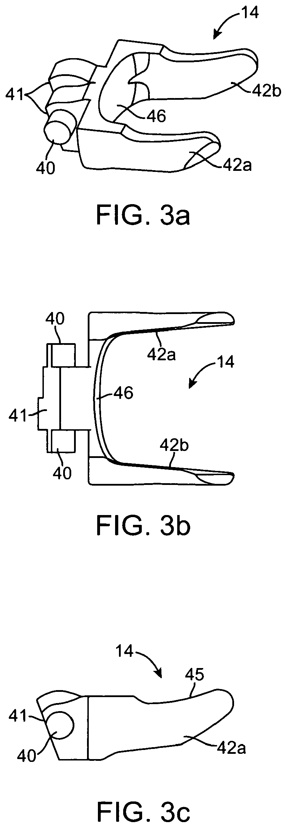

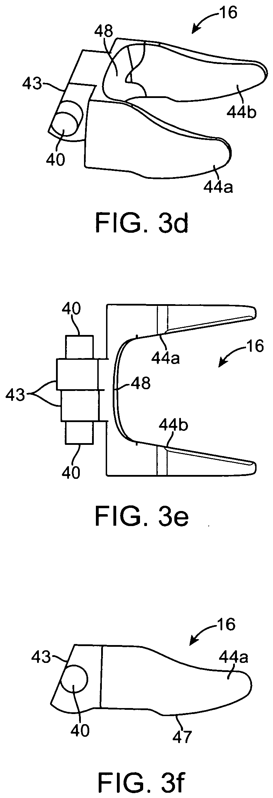

Turning now to FIGs. 3a-3c, the superior arm 14 is shown and in FIGs. 3d-3f, the inferior arm 16 is shown. The superior and inferior arms 14, 16 include pins 40 for mating with the body 12, in particular, for mating with the slots/openings 28 of the arm receiving portion 24. Each of the superior and inferior arms 14, 16 includes at least one caming surface 41, 43, respectively, for contact with the actuator assembly 18. The superior and inferior arms 14, 16 include elongated superior extensions 42a, 42b and elongated inferior extensions 44a, 44b, respectively. Extensions 42a and 44a are located on the left adjacent to the left body piece 20 and extensions 42b and 44b are located on right adjacent to the right body piece 22. Superior extensions 42a, 42b extend substantially parallel to each other in both an undeployed configuration and in a deployed configuration as do inferior extensions 44a, 44b. Extending between extensions 42a, 42b is a strut, bridge, bracket or saddle 46 that forms a superior substantially U-shaped configuration that is sized and configured to receive a superior spinous process. As seen in FIG. 3c, the anterior face of the superior extensions 14 includes a slight concavity or curvature 45 for conforming to the bony anatomy of the superior spinous process and or lamina. Extending between inferior extensions 44a, 44b is a strut, bridge, bracket or saddle 48 that forms an inferior substantially U-shaped configuration together with the extensions 44a, 44b that is sized and configured to receive an inferior spinous process of a spinal motion segment. As seen in FIG. 3f, the anterior face of the inferior extensions 16 includes a slight convexity or curvature 47 for conforming to the bony anatomy of the inferior spinous process and/or lamina. In one variation, the length of the saddle 46 of the superior arm 14 is approximately 8.5 millimeters and the length of the saddle 48 of the inferior arm 16 is approximately 6.6 millimeters. Also, the tip-to-tip distance of the superior extensions 42a, 42b is approximately 9.8 millimeters and the tip-to-tip distance of the inferior extensions 44a, 44b is approximately 9.4 millimeters. In sum, the seat comprising the saddle 46 and superior extensions 42a, 42b formed by the superior arm 14 is larger than the seat comprising the saddle 48 and inferior extensions 44a, 44b formed by the inferior arm 16. The larger superior seat of the spacer conforms closely to a wider lower end of the spinous process and the smaller inferior seat of the spacer conforms closely to a narrower upper end of the adjacent inferior spinous process when the spacer 10 is inserted between adjacent spinous processes as spinous processes are naturally narrower on top and wider on the bottom.

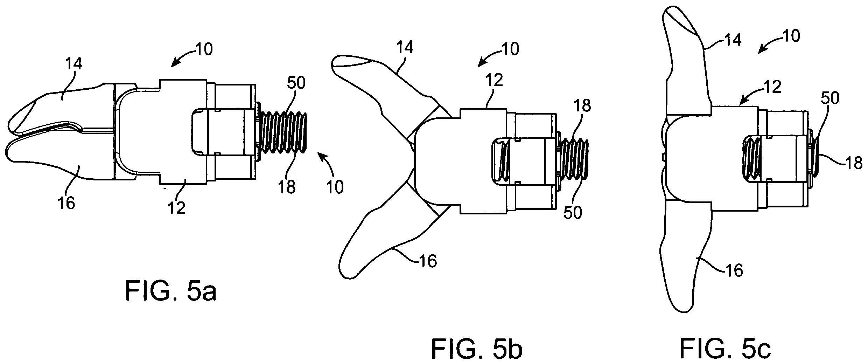

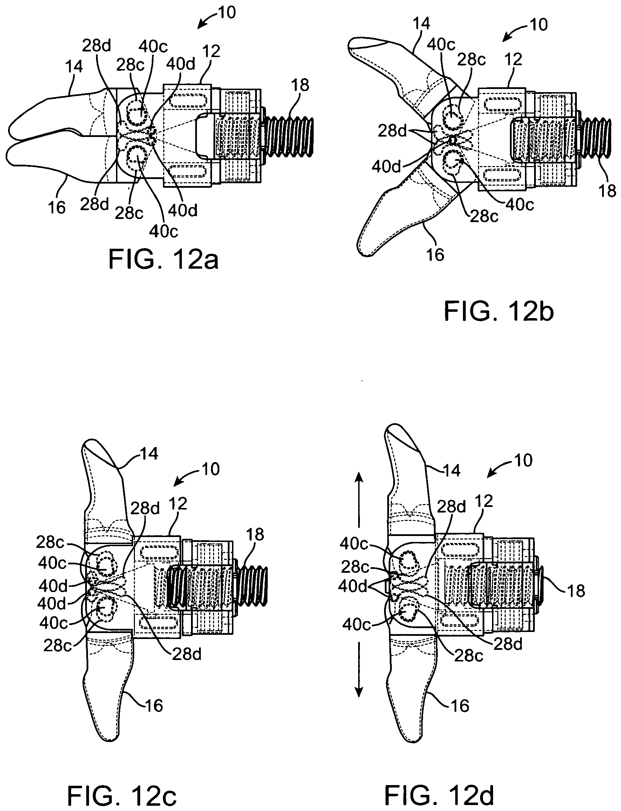

The superior and inferior arms 14, 16 are movably or rotatably connected to the body 12, for example by hinge means or the like to provide rotational movement from an undeployed configuration to a deployed configuration that arcs through about a 90 degree range or more with respect to the body 12. The arms 14, 16 are rotationally movable between at least an undeployed, collapsed or folded state (as shown in FIGs. 1a-1e, 5a, 6a and 7a) and at least one deployed state (as shown in FIGs. 5c, 6c, 7c). In the undeployed state, the arm pairs 14, 16 are aligned generally or substantially axially (i.e., axially with the longitudinal axis, defined by the body 12 or to the translation path into the interspinous space of the patient) to provide a minimal lateral or radial profile. The longitudinal axis X of the spacer 10 and body 12 is shown in FIG. 1c. In the deployed state, the arm pairs 14, 16 are positioned such that each of the U-shaped saddles are in a plane (or planes) or have a U-shaped projection in a plane that is (are) generally or substantially transverse to the longitudinal axis X defined by the body 12 or to the collapsed position or to the implantation path into the interspinous space of the patient. In one variation, the spacer 10 is configured such that the arms 14, 16 are linearly moveable or translatable within the same transverse plane from a first deployed state (such as the state shown in FIG. 12c) to and from a second deployed state (such as the state shown in FIG. 12d) characterized by an additional translation of at least one of the arms 14, 16 with respect to the body 12 along a direction of the arrows as shown in FIG. 12d away from or towards the body 12. The arms 14, 16 can be extended in the general vertical direction along an axis along the general length of the spine wherein the arms 14, 16 are extended away from each other and away from the body 12 as denoted by the arrows in FIG. 12d. The arms 14, 16 can be un-extended in a direction towards each other and towards the body 12 for un-deployment or repositioning of the spacer 10. This feature advantageously allows for the most minimally invasive configuration for the spacer without compromising the ability of the spacer 10 to seat and contain the spinous processes in between levels where the anatomy of the spinous processes is such that the interspinous process space increases in the anterior direction or without compromising the ability of the spacer to provide adequate distraction. The arms 14, 16 are connected to the body 12 and/or to each other in a manner that enables them to be moved simultaneously or independently of each other, as well as in a manner that provides passive deployment and/or vertical extension or, alternatively, active or actuated deployment and/or vertical extension.

Turning back to FIG. 1f, the actuator assembly 18 will now be described. The actuator assembly 18 includes an actuator 48 connected to a shaft 50 and retainer 52, a spindle 86 and a optional lock 88. The actuator 48 includes a distal end 54 and a proximal end 56 and at least two bearing surfaces 58. The bearing surfaces 58 angle towards each other from the proximal end 56 to the distal end 54. In one variation as shown in FIG. 1f, the actuator 48 is integrally formed with the shaft 50. The shaft 50 is substantially cylindrical in shape and includes a threaded outer surface for engagement with a threaded inner surface of the spindle 86. The distal end 54 of the actuator 48 is further configured to engage the superior and inferior arms 14, 16 such that forward translation of the actuator 48 relative to the body 12 effects deployment of the arms into at least one deployed configuration.

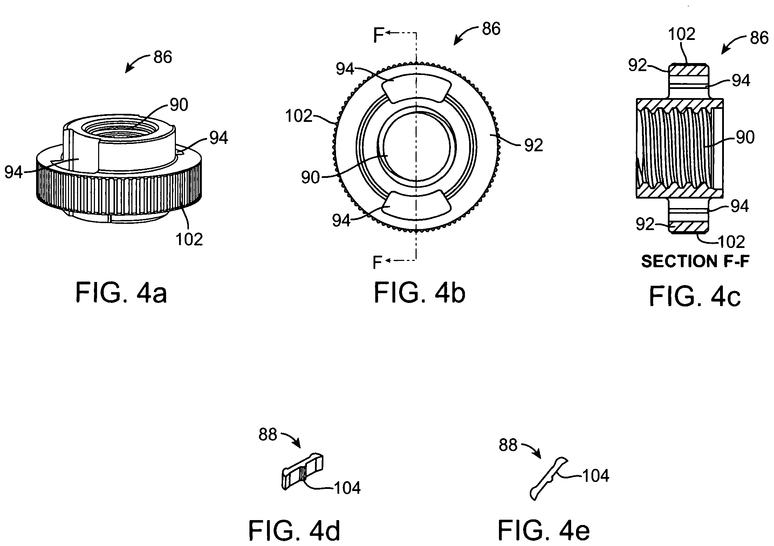

Still referencing FIG. 1f and with particular reference to FIGs. 4a-4c, the spindle 86 has circular top profile and includes a central bore 90 having a threaded inner surface which is sized for threaded connection to the shaft 50. The spindle 86 includes an outer ledge 92 and oppositely disposed notches 94 for connecting to a deployment instrument. The outer sidewall of the spindle 86 includes a plurality of spindle teeth 102. The spindle 86 is configured to be disposed in the spindle receiving portion 80 of the body 12.

Still referencing FIG. 1f, the retainer 52, which is preferably made of metal such as surgical steel or titanium, includes a proximal end 70 and at least one prong 72 extending distally from the proximal end 70. Each prong 72 includes a hook portion 96 for hooking to the undercut 98 of the body 12 to attach the retainer 52 to the body 12. Each prong 72 is allowed to deflect and spring back to snap engage the undercut 98 and thereby connect to the body 12 and retain the actuator assembly 18 to the body 12. An aperture 100 is sized for clear passage of the actuator 48 and shaft 50. The actuator assembly 18 is at least partially disposed inside the body 12 and is configured to move with respect to the body 12.

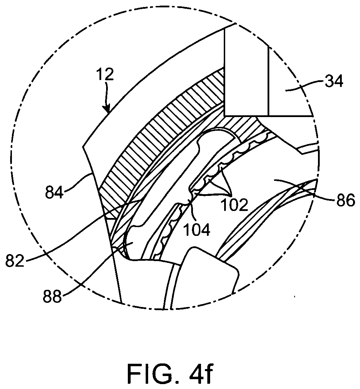

Still referencing FIG. 1f and with particular reference to FIGs. 4d, 4e and 4f, the lock 88 is a small elongate piece of metal or other suitable material such as steel or titanium capable of deflection. The lock 88 is sized to be disposed in the lock receiving portion 82 as shown in FIG. 4f. The lock 88 includes a tooth 104 which is configured to engage the spindle teeth 102. Rotation of the spindle 86 deflects the lock 88 outwardly which then snaps back into between the spindle teeth 102 to lock the spindle 86 in place.

Assembly of the spacer 10 with reference to FIGs. 1a-1f will now be described. The arms 14, 16 are disposed in the arm receiving portion 24 of one body piece. The other of the left or right body piece 20, 22 is securely connected/welded to the one body piece thereby capturing the arms 14, 16 inside the arm receiving portion 24 such that the arms 14, 16 are capable of at least rotational movement with respect to the body 12 and in one variation, capable of rotational movement and translation with respect to the body 12. In a variation in which the body 12 is made of one piece, the arms 14, 16 are movably connected to the body 12 with a pin, for example. The shaft 50 and the actuator 48 are together inserted into the proximal opening 36 and passageway 30 of the body 12. The spindle 86 is disposed in the spindle receiving portion 80 of the body 12 and threaded onto the shaft 50. The lock 88 is disposed inside the lock receiving portion 82 of the body 12. The retainer 52 is connected to the body 12 such that the hooked portion(s) 96 snap into the undercut(s) 98 and such that the shaft 50 can pass through the retainer aperture 100. The retainer 52 captures the spindle 86, actuator 48, shaft 50 and lock 88 inside the body 12 such that the spindle 86 is allowed to rotate and, thereby, move the actuator and shaft 48, 50 inside the body passageway 30.

Referring now to FIGs. 5a-5d, the spacer 10 is shown in a closed, undeployed configuration (FIG. 5a), a partially deployed configuration or otherwise intermediary configuration (FIG. 5b), and a deployed configuration (FIG. 5c). In moving from an undeployed to a deployed configuration, the actuator assembly 18 and, in particular, the shaft 50 of the actuator assembly moves distally with respect to the body to a position flush or almost flush with the proximal end of the body 12 or to a position completely inside the body 12 disappearing from sight providing a low profile for the spacer 10 along the longitudinal axis of the body 12.

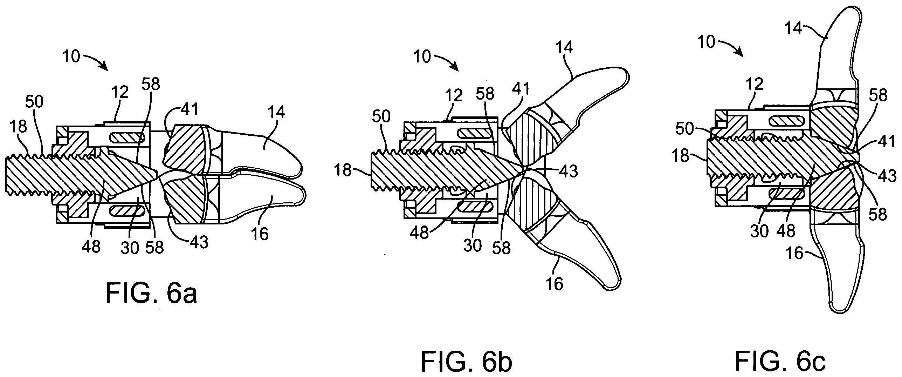

Turning now to the cross-sectional views of the spacer 10 in FIGs. 6a-6c, as the shaft 50 advances within the passageway 30, the bearing surfaces 58 of the actuator 48 contact the superior and inferior caming surfaces 41, 43 of the superior and inferior arms 14, 16 turning the arms 14, 16 into rotation with respect to the body 12. Upon rotation, the bearing surfaces 58 of the actuator 48 slide with respect to the superior and inferior caming surfaces 41, 43 of the superior and inferior arms 14, 16. The arms 14, 16 rotate through an arc of approximately 90 degrees with respect to the body 12 into the deployed configuration (FIG. 6c) in which the superior and inferior extensions of the arms 14, 16 are substantially perpendicular to the longitudinal axis of the spacer 10 as shown in FIGs. 6c. The arms 14, 16 have a substantially U-shaped projection in a plane perpendicular to the longitudinal axis of the spacer 10.

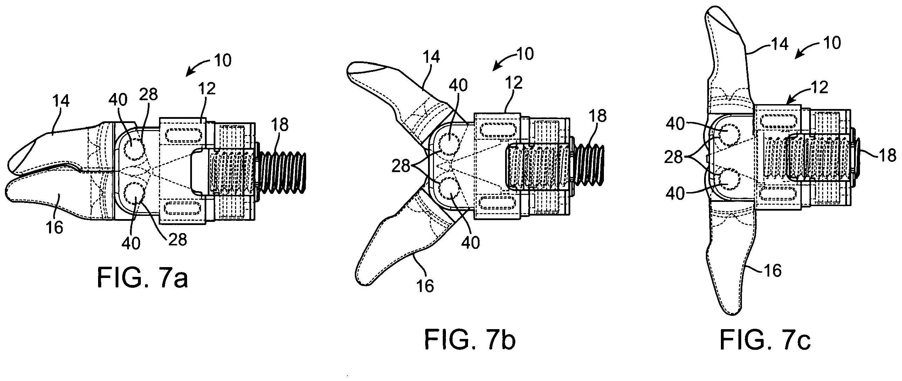

Turning now to the semi-transparent views of the spacer 10 in FIGs. 7a-7c, the rotation of the pins 40 of the arms 14, 16 in the openings 28 of the body 12 is shown in moving from the configuration of FIG. 7a to the configuration of FIG. 7c. Reverse rotation of the spindle 86 moves the shaft 50 proximally with respect to the body 12 allowing the arms to close to any intermediary configuration between a deployed, configuration and an undeployed, closed configuration. This feature advantageously permits the surgeon to ease installation and positioning of the spacer with respect to patient anatomy.

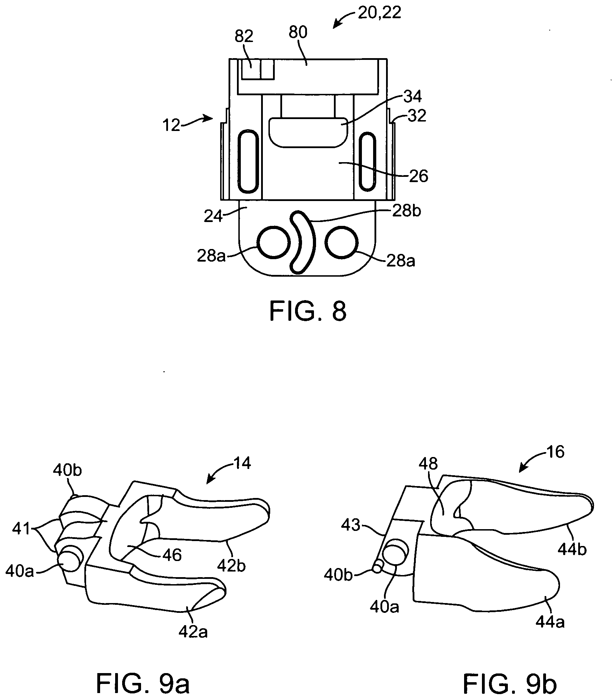

Turning now to FIG. 8, another variation of the body 12 will now be discussed wherein like reference numbers are used to describe like parts. The body 12 of the variation shown in FIG. 8 has the same clamshell construction with the left body piece 20 joined to a right body piece 22 to capture arms 14, 16 inside. With the right and left body pieces 20, 22 joined together, the body 12 is generally cylindrical. It has a cross-sectional size and shape that allows for implantation between adjacent spinous processes and facilitates delivery into a patient through a narrow port or cannula. The left and right body pieces 20, 22 are identical and therefore, FIG. 8 illustrates either the left or right body piece 20, 22.

Still referencing FIG. 8, the inside of the body 12 defines an arm receiving portion 24 and an actuator assembly receiving portion 26 with features formed in each of the left and right body pieces 20, 22 that together define the arm and actuator assembly receiving portions 24, 26. The arm receiving portion 24 includes slots or openings or apertures 28 that receive pins formed on the arms 14, 16 such that the pins rotate and/or translate inside the slots or apertures 28. In the variation shown in FIG. 8, in addition to two circular openings 28a, there is provided a curved slot 28b in each of the left and right body pieces 20, 22. The circular openings 28a and curved slot 28b are configured to receive pins 40 of arms 14, 16 that are illustrated in FIGs. 9a and 9b.

Turning now to FIGs. 9a and 9b, the superior arm 14 is shown in FIG. 9a, and the inferior arm 16 is shown in FIG. 9b. The superior and inferior arms 14, 16, include pins 40a and 40b for mating with the body 12, in particular, for mating with the openings 28a and slots 28b, respectively. Each side of the superior and inferior arms 14, 16 includes a circular first pin 40a configured for insertion into opening 28a such that the arms 14, 16 rotate with respect to the body 12. At least one side of each of the arms 14, 16 includes a second pin 40b configured for insertion into opening slot 28b. Slot 28b and pin 40b serve as a stop mechanism such that the rotation of the arms 14, 16 with respect to the body 12 is limited by pin 40b in slot 28b. While being deployed, the arms 14, 16 rotate to a position transverse to the longitudinal axis from a position parallel to the longitudinal axis wherein such rotation is arrested by pin 40b abutting the end of slot 28b. Other features of the arms 14, 16 shown in FIGs. 9a and 9b are substantially the same as described above and like reference numbers are used to describe the like parts.

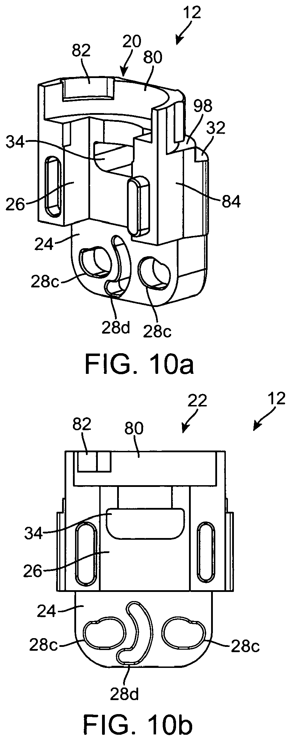

Turning now to FIGs. 10a and 10b, another variation of the spacer body 12 will now be discussed wherein like reference numbers are used to describe like parts. The body 12 of the spacer variation shown in FIGs. 10a and 10b has a clamshell construction as described above with the left body piece 20 joined to a right body piece 22 to capture arms 14, 16 inside. With the right and left body pieces 20, 22 joined together, the body 12 is generally cylindrical. It has a cross-sectional size and shape that allows for implantation between adjacent spinous processes and facilitates delivery into a patient through a narrow port or cannula. FIG. 10a shows the left body piece 20 and FIG. 10b shows the right body piece 22, however, the left and right body pieces 20, 22 are identical.

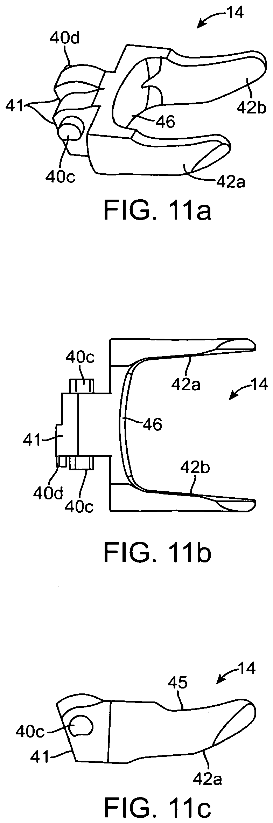

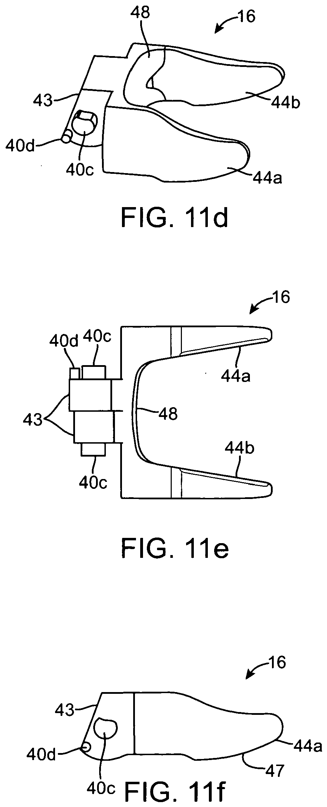

Still referencing FIGs. 10a and 10c, the inside of the body 12, formed by the conjunction of the left and right body pieces 20, 22, defines an arm receiving portion 24 and an actuator assembly receiving portion 26 with features formed in each of the left and right body pieces 20, 22 that together define the arm and actuator assembly receiving portions 24, 26. The arm receiving portion 24 includes slots or openings or apertures 28 that receive pins formed on the arms 14, 16 such that the pins rotate and/or translate inside the slots or apertures 28. In particular, in the variation shown in FIGs. 10a and 10b, two elongated openings 28c and a curved slot opening 28d are provided in each of the left and right body pieces 20, 22. The elongated openings 28c and curved opening 28d are configured to receive pins 40 of arms 14, 16 and serve as channels in which pins 40 can move. The curved slot 28d includes a straight distal portion for translating and extending the arms 14, 16 with respect to the body. Arms 14 and 16 with pins 40 configured to correspond to the left and right body pieces 20, 22 are shown in FIGs. 11a-11f.

Turning now to FIGs. 11a-11f, the superior arm 14 is shown in FIGs. 11a-11c, and the inferior arm 16 is shown in FIGs. 11d-11f. The superior and inferior arms 14, 16, include pins 40c and 40d for mating with the body 12, in particular, for mating with the elongated openings 28c and curved slots 28d, respectively. Each side of the superior and inferior arms 14, 16 includes at least a first pin 40c configured for insertion into opening 28c such that the arms 14, 16 rotate with respect to the body 12 as well as translate with respect to the body 12. At least one side of each of the arms 14, 16 includes a second pin 40d configured for insertion into curved slot 28d such that the arms 14, 16 rotate with respect to the body 12 as well as translate with respect to the body 12. Slots 28d and openings 28c guide the movement of pins 40d and 40c, respectively therein as will be described with respect to FIGs. 12a-12d. Other features of the arms 14, 16 shown in FIGs. 11a-11f are substantially the same as described above and like reference numbers are used to describe the like parts.

Referring now to FIGs. 12a-12d, the spacer 10 is shown in a closed, undeployed configuration (FIG. 12a), a partially deployed or otherwise intermediary configuration (FIG. 12b), a deployed configuration (FIG. 12c), and a deployed and extended configuration (FIG. 12d). In moving from an undeployed to a deployed configuration, the semi-transparent views of the spacer 10 in FIGs. 12a-12d show the rotation and translation of the pins 40 of the arms 14, 16 in the slots 28 of the body 12. The translation of the pins 40 of the arms 14, 16 in the slots 28 of the body 12 is shown in moving from the first deployed configuration of FIG. 12c to the second deployed, extended configuration of FIG. 12d wherein the extension of the arms 14, 16 is in the direction of the arrows in FIG. 12d. Such outward translation with respect to the body 12 is guided by the length and shape of the slots 28. Opening 28c is elongated and slot 28d includes a straight distal end configured to accommodate and guide the extension of arms 14, 16 away from the body 12. Reverse rotation of the spindle 86 moves the shaft 50 proximally with respect to the body 12 allowing the arms 14, 16 to close to any intermediary configuration between a deployed, extended configuration and an undeployed, closed configuration. This feature advantageously permits the surgeon to ease installation and positioning of the spacer with respect to patient anatomy as the arms 14, 16 can be deployed, undeployed and then re-deployed as often as necessary to position the spacer 10.

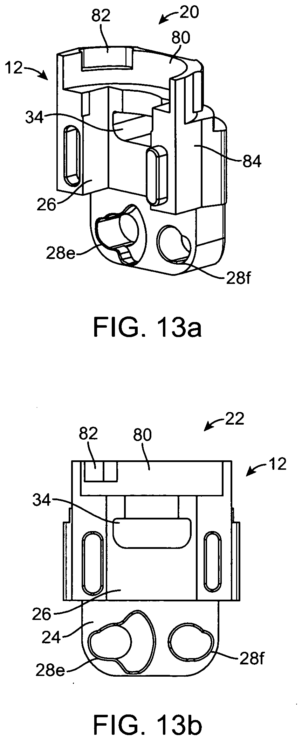

Turning now to FIGs. 13a and 13b, another variation of the spacer with yet another body 12 configuration will now be discussed wherein like reference numbers are used to describe like parts. The body 12 of the variation shown in FIGs. 13a and 13b has a clamshell construction as described above with a left body piece 20 joined to a right body piece 22 to capture arms 14, 16 inside. With the right and left body pieces 20, 22 joined together, the body 12 is generally cylindrical. It has a cross-sectional size and shape that allows for implantation between adjacent spinous processes and facilitates delivery into a patient through a narrow port or cannula. FIG. 13a shows the left body piece 20 and FIG. 13b shows the right body piece 22, however, the left and right body pieces 20, 22 are identical.

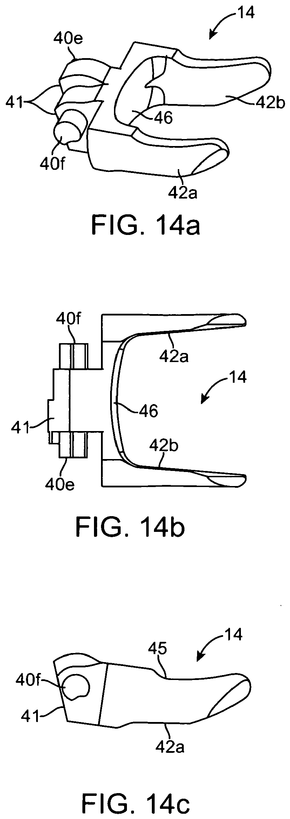

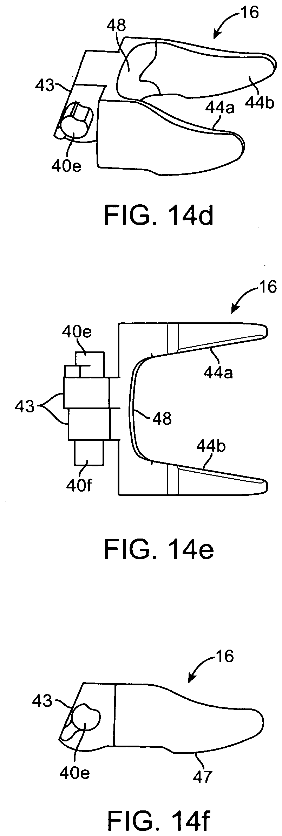

Still referencing FIGs. 13a and 13b, the inside of the body 12, formed by the conjunction of the left and right body pieces 20, 22, defines an arm receiving portion 24 and an actuator assembly receiving portion 26 that includes a spindle receiving portion 80 and lock receiving portion 82 with features formed in each of the left and right body pieces 20, 22 that together define the arm and actuator assembly receiving portions 24, 26. The arm receiving portion 24 includes slots or openings or apertures 28 that receive pins formed on the arms 14, 16 such that the pins rotate and/or translate inside the slots or apertures 28. In particular, in the variation shown in FIGs. 13a and 13b, a first opening 28e and a second opening 28f are provided in each of the left and right body pieces 20, 22. The first opening 28e includes a fanned recess. Both openings 28e and curved slot 28f are configured to receive pins 40 of arms 14, 16 and serve as channels that constrain the movement of the pins 40. In the variation shown, the openings 28e, 28f are configured to permit extension of the arms 14, 16 away from the body. Arms 14, 16 with pins 40 that are configured to correspond to the left and right body pieces 20, 22 are shown in FIGs. 14a-14f.

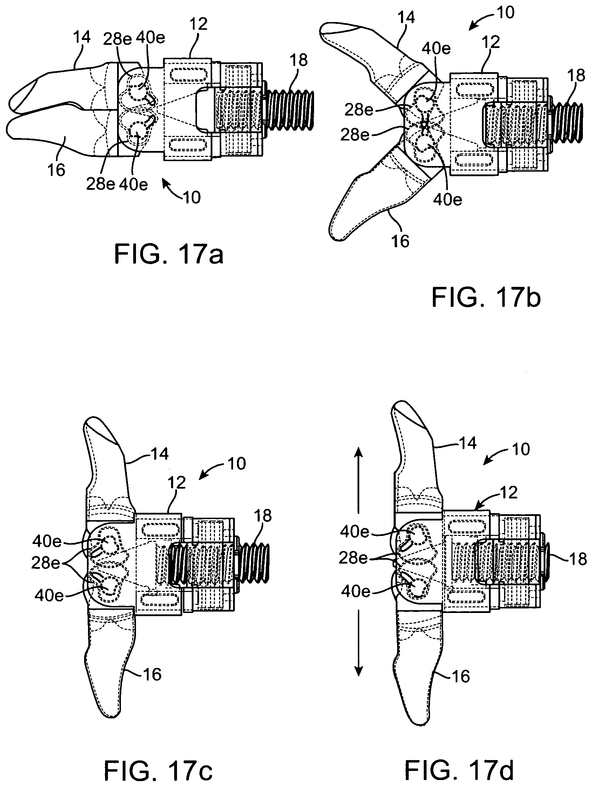

Turning now to FIGs. 14a-14f, the superior arm 14 is shown in FIGs. 14a-14c, and the inferior arm 16 is shown in FIGs. 14d-14f. The superior and inferior arms 14, 16, include a first pin 40e and a second pin 40f for mating with the body 12, in particular, for mating with the first opening 28e and second opening 28f, respectively. At least one side of each of the superior and inferior arms 14, 16 includes a first pin 40e configured for insertion into opening 28e such that the arms 14, 16 rotate with respect to the body 12 as well as translate with respect to the body 12. At least the other side of each of the arms 14, 16 includes a second pin 40f configured for insertion into curved slot 28f such that the arms 14, 16 rotate with respect to the body 12 as well as translate with respect to the body 12. The first pin 40e includes a central portion integrally formed with a peripheral or projecting portion in what resembles a merging of two pins into one larger pin. This larger pin 40e advantageously provides a larger bearing surface capable of bearing larger loads in arresting rotation of the arms 14, 16. The first and second openings 28e, 28f guide the movement of pins 40e and 40f, respectively as will be described with respect to FIGs. 17a-17d. Other features of the arms 14, 16 shown in FIGs. 14a-14f are substantially the same as described above and like reference numbers are used to describe the like parts.

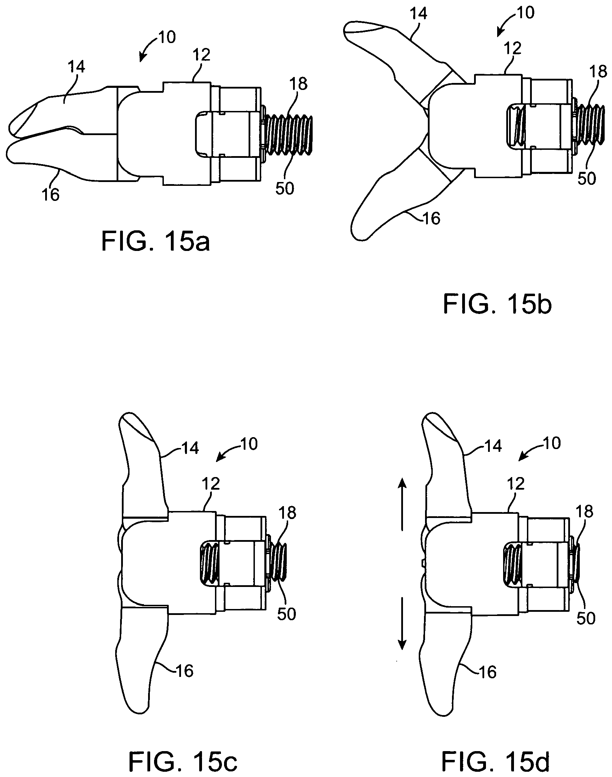

Referring now to FIGs. 15a-15d, the spacer 10 having a body 12 of FIGs. 13a and 13b is shown in a closed, undeployed configuration (FIG. 15a), a partially deployed or otherwise intermediary configuration (FIG. 15b), a deployed configuration (FIG. 15c), and a deployed and extended configuration (FIG. 15d).

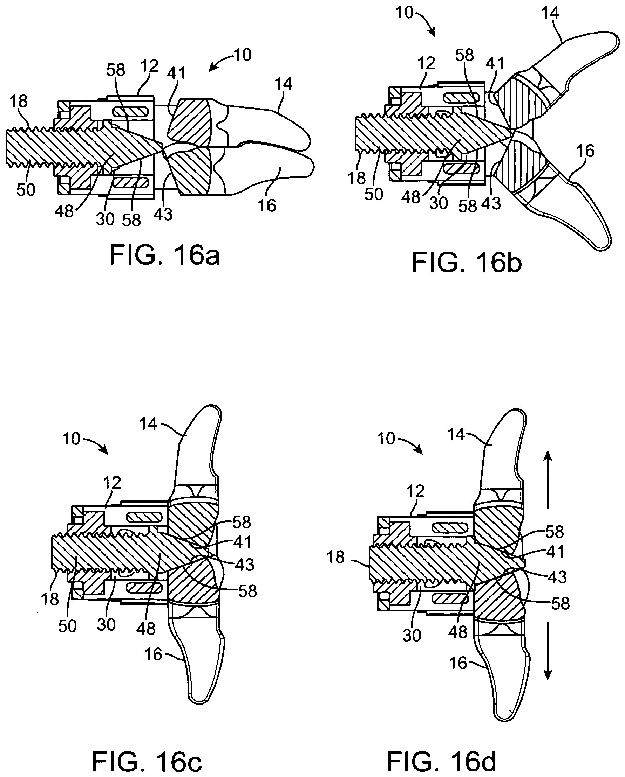

Turning now to the cross-sectional views of the spacer 10 in FIGs. 16a-16d, as the spindle 86 is rotated and the shaft 50 advances within the passageway 30, the bearing surfaces 58 of the actuator 48 contact the superior and inferior caming surfaces 41, 43 of the superior and inferior arms 14, 16 turning the arms 14, 16 into rotation with respect to the body 12. Upon rotation, the bearing surfaces 58 of the actuator 48 slide with respect to the superior and inferior caming surfaces 41, 43 of the superior and inferior arms 14, 16. The arms 14, 16 rotate through an arc of approximately 90 degrees with respect to the body 12 into the deployed configuration (FIG. 16c) in which the superior and inferior extensions of the arms 14, 16 are substantially perpendicular to the longitudinal axis of the spacer 10 as shown in FIGs. 16c and with further actuation, into a deployed and extended configuration (FIG. 16d) in which the superior and inferior extensions of the arms 14, 16 are substantially perpendicular to the longitudinal axis of the spacer 10 and the arms 14, 16 are moved away from the body 12 in a transverse direction to the longitudinal axis as shown by the arrows in FIG. 16d.

Turning now to FIGs. 17a-17d, semi-transparent views of the spacer 10 are shown. In moving from an undeployed to a deployed configuration, the rotation and translation of the pins 40e, 40f of the arms 14, 16 in the slots 28e, 28f of the body 12 is shown. Following rotation, the translation of the pins 40e, 40f of the arms 14, 16 in the slots 28e, 28f, respectively, is shown in moving from the first deployed configuration of FIG. 17c to the second deployed, extended configuration of FIG. 17d in the direction of the arrows in FIG. 17d. Such outward translation with respect to the body 12 is guided by the length and shape of the slots 28e, 28f. Reverse rotation of the spindle 86 moves the shaft 50 proximally with respect to the body 12 allowing the arms to close to any intermediary configuration between a deployed, extended configuration and an undeployed, closed configuration. This feature advantageously permits the surgeon to ease installation and positioning of the spacer with respect to patient anatomy.

To deliver and deploy the spacer 10 within the patient, the spacer 10 is releasably attached to an insertion instrument 80 at the proximal end of the spacer 10 via notches 34. The insertion instrument 80 includes a first assembly 102 connected to a second assembly 104 and a handle assembly 106.

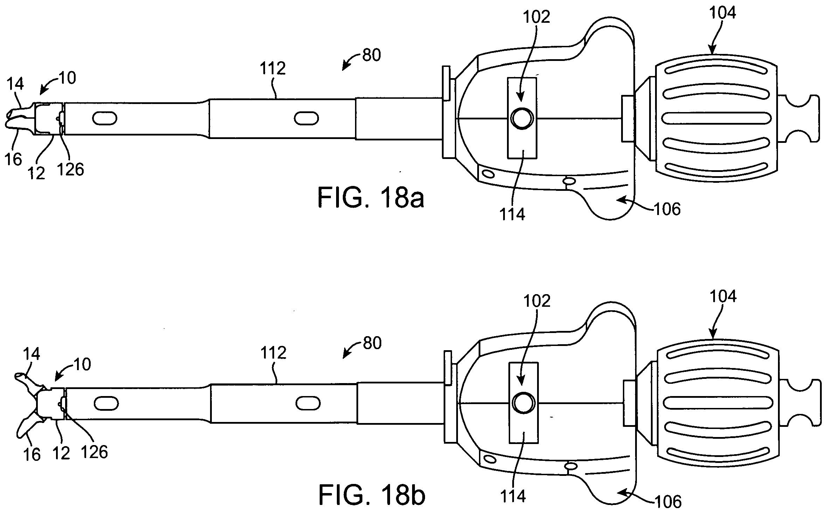

The spacer 10 is provided or otherwise placed in its undeployed, closed state in juxtaposition to the insertion instrument 80 and connected thereto as shown in FIG. 18a. The longitudinal axis of the insertion instrument 80 is advantageously aligned with the longitudinal axis of the spacer 10 as shown. The delivery instrument 80 includes a first subassembly 102 to releasably clamp to the body 12 of the spacer 10 at a distal end of the insertion instrument 80. The first subassembly 102 includes an inner clamp shaft (not shown) having flexible prongs 126 at the distal end configured for attachment to the body 12 of the spacer 10 and, in particular, for insertion into the notches 34 of the spacer body 12. The first subassembly 102 includes an outer shaft 112 located over the inner clamp shaft and configured for relative motion with respect to one another via a control 114 located at the handle assembly 106. The control 114 is threaded to the outer shaft 112 such that rotation of the control 114 moves the outer shaft 112 along the longitudinal axis of the insertion instrument 80 over the inner clamp shaft to deflect and undeflect the prongs 126 to connect or disconnect the instrument 80 to or from the body 12. The first control 114 is activated at the handle of the insertion instrument 100 such that the first subassembly 102 is connected to the body 12 of the spacer 10. The first control 114 is rotated in one direction to advance the outer shaft 112 over the inner clamp shaft (not shown) deflecting the prongs 118 inwardly into the notches 34 on the body 12 of the spacer 10 to secure the spacer body 12 to the instrument as shown in FIG. 18a. Reverse rotation of the control 114 reverses the direction of translation of the outer shaft 112 to release the prongs 126 from the notches 34 and, thereby, release the spacer 10 from the instrument 80.

Still referencing FIG. 18a, the insertion instrument 80 includes a second subassembly 104 that is configured to connect to the actuator assembly 18 of the spacer 10. In particular, the second subassembly 104 includes means located at the distal end of the second subassembly 104 to activate the actuator assembly 18. In one variation, the second subassembly 104 is a pronged driver having an elongated shaft that is configured to be insertable into the notches 94 of the spindle 86 while the spacer 10 is connected to the instrument 80. As seen in FIG. 4b, there are two notches 94 oppositely located from each other in the spindle 86. The distal end of the driver includes prongs that correspond to the notches 94 and configured to be inserted into the notches 94. The second subassembly 104 is insertable at the proximal end of the instrument 80 and extends through the handle assembly 106 and through the inner shaft until the notches are engaged by the distal end. The removable driver 104 is rotatable with respect to the instrument 80 to rotate the spindle 86 and arrange the spacer 10 to and from deployed and undeployed configurations.

To deliver and deploy the spacer 10 within the patient, the spacer 10 is releasably attached to a delivery instrument 80 at the proximal end of the spacer 10 as described. A small midline or lateral-to-midline incision is made in the patient for minimally-invasive percutaneous delivery. In one variation, the supraspinous ligament is avoided. In another variation, the supraspinous ligament is split longitudinally along the direction of the tissue fibers to create an opening for the instrument. Dilators may be further employed to create the opening. In the undeployed state with the arms 14, 16 in a closed orientation and attached to a delivery instrument 80, the spacer 10 is inserted into a port or cannula, if one is employed, which has been operatively positioned to an interspinous space within a patient's back and the spacer is passed through the cannula to the interspinous space between two adjacent vertebral bodies. The spacer 10 is advanced beyond the end of the cannula or, alternatively, the cannula is pulled proximately to uncover the spacer 10 connected to the instrument 80. Once in position, the second assembly 104 is inserted into the instrument 80 if not previously inserted to engage the spindle notches 94 and is rotated to rotate the spindle 86. The rotating spindle 86 then advances the actuator 48 and shaft 50 to begin deployment the spacer 10. Rotation in one direction, clockwise, for example, threadingly advances the shaft 50 through the spindle central bore 90 which then results in the actuator 48 contacting the superior and inferior caming surfaces 41, 43 of the superior and inferior arms 14, 16 to begin their deployment. FIG. 18b illustrates the superior arm 14 and the inferior arm 16 in a partially deployed position with the arms 14, 16 rotated away from the longitudinal axis. Rotation of the driver 104 turns the spindle 86 which in turn rotates the actuator shaft 50 threadingly advancing it with respect to the body 12 which distally advances the actuator 48 whose bearing surfaces 58 contact the superior and inferior camming surfaces 41, 43 pushing the superior and inferior arms 14, 16 into rotation about the pins 40 that are guided in the openings 28. The lock 88 snaps into the spindle teeth 102 advantageously locking the deployment of the arms at any degree of rotation of the spindle 86 to prevent the arms 14, 16 from folding and providing a tactile and audio feedback of the deployment progress. The lock 88 permits further rotation or de-rotation as desired.

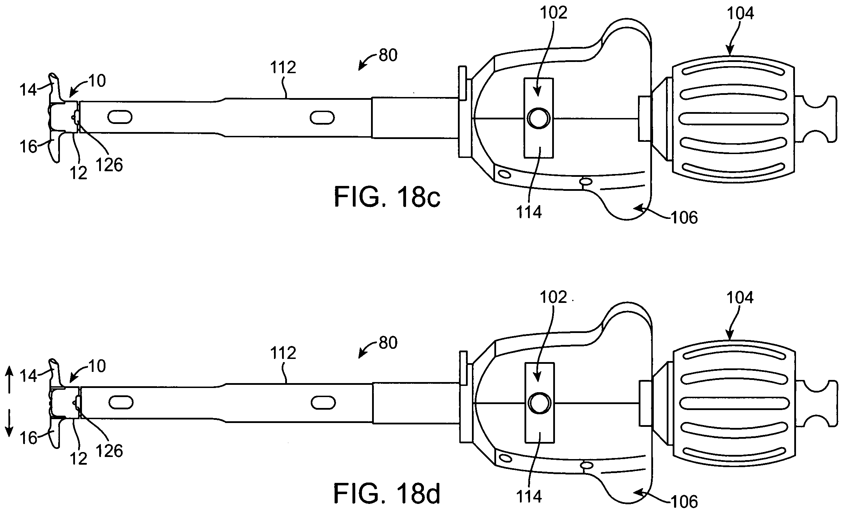

The position of the arms 14, 16 in FIG. 18b may be considered to be one of many partially deployed configurations or intermediary configurations that are possible and from which the deployment of the arms 14, 16 is reversible with opposite rotation of the second assembly 104. With further advancement, the arms 14, 16 rotate through an arc of approximately 90 degrees into the deployed configuration in which the superior and inferior extensions are substantially perpendicular to the longitudinal axis of the spacer 10 as shown in FIG. 18c.

Turning to FIG. 18c, there is shown an insertion instrument 80 connected to a spacer 10 in a first deployed configuration in which the arms 14, 16 are approximately 90 degrees perpendicular to the longitudinal axis or perpendicular to the initial undeployed configuration. Continued rotation of second assembly 104 rotates the spindle 86 and threads the shaft 50 further distally with respect to the body 12 of the spacer 10 pushing the bearing surfaces 58 further against the superior and inferior camming surfaces 41, 43. While in the first deployed configuration of FIG. 18c, the clinician can observe with fluoroscopy the positioning of the spacer 10 inside the patient and then choose to reposition the spacer 10 if desired. Repositioning of the spacer may involve undeploying the arms 14, 16 by rotating the spindle 86 via the second assembly 104 to rotate the arms into any one of the many undeployed configurations. The spacer may then be re-deployed into the desired location. This process can be repeated as necessary until the clinician has achieved the desired positioning of the spacer in the patient. Of course, inspection of the spacer 10 may be made via fluoroscopy while the spacer 10 is in an intermediate or partially deployed configuration such as that of FIG. 18b.

Even further advancement of the actuator shaft 50 via rotation of the second subassembly 104 from the first deployed configuration results in the spacer 10 assuming a second deployed configuration shown in FIG. 18d, if the spacer 10 is so configured as to allow a second deployed configuration. The second deployed configuration is an extended configuration as described above in which the superior and inferior arms 14, 16 extend transversely with respect to the longitudinal axis outwardly in the direction of the arrows in FIG. 18d. The spacer 10 is configured such that the outward translation of the arms 14, 16 follows the rotation into 90 degrees and is guided by the length and shape of the openings 28 in which the arms 14, 16 move. Once deployed, the superior arm 14 seats the superior spinous process and the inferior arm 16 seats the adjacent inferior spinous process. Such extension may also provide some distraction of the vertebral bodies.

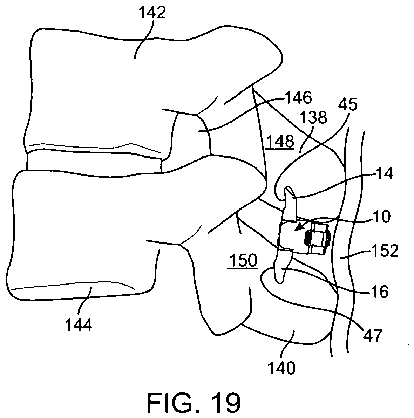

Following deployment, the second assembly 104 may be removed. Control 114 is rotated in the opposite direction to release the body 12 from the instrument 80. The insertion instrument 80, thus released from the spacer 10, is removed from the patient leaving the spacer 10 implanted in the interspinous process space as shown in FIG. 19. In FIG. 19, the spacer 10 is shown with the superior arm 14 seating the superior spinous process 138 of a first vertebral body 142 and the inferior arm 16 seating the inferior spinous process 140 of an adjacent second vertebral body 144 providing sufficient distraction to open the neural foramen 146 to relieve pain. As mentioned above, the shape of the superior arm 14 is such that a superior concavity or curvature 45 is provided to conform to the widening of the superior spinous process 138 in an anterior direction toward the superior lamina 148 going in the anterior direction. In general, the superior arm 14 is shaped to conform to anatomy in the location in which it is seated. Likewise, as mentioned above, the shape of the inferior arm 16 is such that an inferior convexity or curvature 47 is provided to conform to the widening of the inferior spinous process 140 in an anterior direction toward the inferior lamina 150. The supraspinous ligament 152 is also shown in FIG. 19.

The spacer 10 is as easily and quickly removed from body of the patient as it is installed. The instrument 80 is inserted into an incision and reconnected to the spacer 10. The shaft 50 is rotated in the opposite direction via a driver 104 to fold the arms 14, 16 into a closed or undeployed configuration. In the undeployed configuration, the spacer 10 can be removed from the patient along with the instrument 80 or, of course, re-adjusted and re-positioned and then re-deployed as needed with the benefit of minimal invasiveness to the patient.

Any of the spacers disclosed herein are configured for implantation employing minimally invasive techniques including through a small percutaneous incision and through the superspinous ligament. Implantation through the superspinous ligament involves selective dissection of the superspinous ligament in which the fibers of the ligament are separated or spread apart from each other in a manner to maintain as much of the ligament intact as possible. This approach avoids crosswise dissection or cutting of the ligament and thereby reduces the healing time and minimizes the amount of instability to the affected spinal segment. While this approach is ideally suited to be performed through a posterior or midline incision, the approach may also be performed through one or more incisions made laterally of the spine with or without affect to the superspinous ligament. Of course, the spacer may also be implanted in a lateral approach that circumvents the superspinous ligament altogether as well as in open or mini-open procedures.

A spacer may include only a single arm which is configured to receive either the superior spinous process or the inferior spinous process. The surface of the spacer body opposite the side of the single arm may be contoured or otherwise configured to engage the opposing spinous process wherein the spacer is sized to be securely positioned in the interspinous space and provide the desired distraction of the spinous processes defining such space. The additional extension of the arm(s) subsequent to their initial deployment in order to seat or to effect the desired distraction between the vertebrae may be accomplished by expanding the body portion of the device instead of or in addition to extending the individual extension members 14, 16.

The extension arms of the subject device may be configured to be selectively movable subsequent to implantation, either to a fixed position prior to closure of the access site or otherwise enabled or allowed to move in response to normal spinal motion exerted on the device after deployment. The deployment angles of the extension arms may range from less than 90 degrees (relative to the longitudinal axis defined by the device body) or may extend beyond 90 degrees and remain stationary or be dynamic. Each extension member may be rotationally movable within a range that is different from that of the other extension members. Additionally, the individual superior and/or inferior extensions 42a, 42b, 44a, 44b may be movable in any direction relative to the strut or bridge extending between an arm pair or relative to the device body in order to provide shock absorption and/or function as a motion limiter, or serve as a lateral adjustment particularly during lateral bending and axial rotation of the spine. The manner of attachment or affixation of the extensions to the arms may be selected so as to provide movement of the extensions that is passive or active or both. In one variation, the saddle or distance between extensions 42a and 42b or between 44a and 44b can be made wider to assist in seating the spinous process and then narrowed to secure the spinous process positioned between extensions 42a and 42b or between 44a and 44b.

The preceding merely illustrates the principles of the invention. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein. Rather, the scope of present invention is embodied by the appended claims.

## Claims

1. An implantable spacer (10) for placement between adjacent spinous processes comprising:
a body (12) defining a longitudinal axis;
a first arm (14) and a second arm (16) connected to the body and capable of movement with respect to the body such that the first and second arms are rotatable away from each other and away from the longitudinal axis of the body;
each arm defining a configuration for receiving a spinous process;
each arm having a proximal caming surface (41,43);
an actuator assembly (18) connected to the body;
the actuator assembly comprising:
an actuator (48) having at least one bearing surface (58);
a threaded shaft (50) connected to the actuator and configured for movement with respect to the body; and
a spindle (86); **characterized in that** the actuator assembly is configured to move relative to the body such that rotation of the spindle moves the actuator such that the at least one bearing surface contacts at least one of the caming surfaces to move both of the arms from an undeployed configuration to a deployed configuration in which the first arm receives and seats a first spinous process and the second arm receives and seats an adjacent second spinous process.

2. The spacer of claim 1 further including a retainer configured to retain the actuator and spindle with respect to the body such that the actuator moves and the spindle rotates relative to the body.

3. The spacer of claims 1-2 wherein the spindle includes an inner threaded surface and the shaft includes an outer threaded surface configured for threaded engagement with each other.

4. The spacer of claims 1-3 further including a lock configured to resist rotation of the spindle.

5. The spacer of claims 1-4 further including a lock having at least one tooth and the spindle further including a plurality of teeth on the other surface configured to engage the at least one tooth on the lock.

6. The spacer of claims 1-5 wherein each of the first and second arms includes at least one pin and the body includes corresponding apertures configured for connecting the first and second arms to the body at the pins such that the arms are movable with respect to the body.

7. The spacer of claims 1-6 wherein the apertures and pins are configured such that the first and second arms are capable of rotation with respect to the body.

8. The spacer of claims 1-6 wherein the apertures and pins are configured such that the first and second arms are capable of rotation and translation with respect to the body.

9. The spacer of claims 1-8 wherein the apertures and pins are configured such that the first and second arms are capable of rotation followed by translation with respect to the body.

10. The spacer of claims 1-9 wherein the first and second arms rotate approximately 90 degrees into at least one deployed configuration in which the arms are approximately transverse to the longitudinal axis of the body; said rotation being from an undeployed configuration in which the arms are substantially parallel to the longitudinal axis of the body.

11. The spacer of claims 1-10 wherein the first and second arms rotate approximately 90 degrees into at least one deployed configuration in which the arms are approximately transverse to the longitudinal axis of the body;
said rotation being from an undeployed configuration in which the arms are substantially parallel to the longitudinal axis of the body; and
wherein following said rotation upon further actuation the arms translate with respect to the body in a direction substantially transverse to longitudinal axis of the body.

12. The spacer of claims 1-11 wherein the body is configured for attachment to a spacer delivery instrument such that the longitudinal axis of the spacer delivery instrument is substantially aligned with the longitudinal axis of the spacer.

13. The spacer of claims 1-12 wherein the spindle includes notches for receiving a tool to rotate to the spindle with respect to the body.

14. The spacer of claims 1-13 wherein rotation of the spindle in one direction advances the actuator distally to engage the caming surfaces and deploy the arms into at least one deployed configuration and rotation of the spindle in an opposite direction moves the actuator proximally to permit the arms to fold into at least one undeployed or intermediary configuration.

15. The spacer of claims 1-14 wherein each arm has a pair of extensions and a saddle defining a configuration that is substantially U-shaped for seating a spinous process.

16. The spacer of claims 1-14 wherein each arm has a pair of extensions, and wherein the extensions of the first arm are spaced apart from one another such that they are able to receive and seat the first spinous process and the extensions of the second arm are spaced apart from one another such that they are able to receive and seat the second spinous process.

## Patentansprüche

1. Implantierbarer Abstandhalter (10) zur Platzierung zwischen benachbarten Dornfortsätzen, umfassend:
einen Körper (12), der eine Längsachse definiert;
einen ersten Arm (14) und einen zweiten Arm (16), die mit dem Körper verbunden und zu einer derartigen Bewegung in Bezug auf den Körper fähig sind, dass der erste und der zweite Arm weg voneinander und weg von der Längsachse des Köpers drehbar sind;
wobei jeder Arm eine Konfiguration zum Aufnehmen eines Dornfortsatzes definiert;
wobei jeder Arm eine proximale Nockenfläche (41, 43) aufweist;
eine Betätigungselementbaugruppe (18), die mit dem Körper (12) verbunden ist; wobei die Betätigungselementbaugruppe umfasst:
ein Betätigungselement (48) mit mindestens einer Lagerfläche (58);
eine Gewindewelle (50), die mit dem Betätigungselement verbunden und zur Bewegung in Bezug auf den Körper konfiguriert ist; und
eine Spindel (86);
**dadurch gekennzeichnet, dass**
die Betätigungselementbaugruppe dafür konfiguriert ist, sich relativ zu dem Körper derart zu bewegen, dass eine Drehung der Spindel das Betätigungselement bewegt, sodass die mindestens eine Lagerfläche mit mindestens einer der Nockenflächen in Kontakt geht, um beide der Arme aus einer Nicht-Einsatzkonfiguration in eine Einsatzkonfiguration zu bewegen, in welcher der erste Arm einen ersten Dornfortsatz aufnimmt und lagert und der zweite Arm einen benachbarten zweiten Dornfortsatz aufnimmt und lagert.

2. Abstandhalter nach Anspruch 1, ferner aufweisend eine Halteeinrichtung, die zu einem derartigen Halten des Betätigungselements und der Spindel in Bezug auf den Körper konfiguriert ist, dass sich das Betätigungselement bewegt und sich die Spindel relativ zu dem Körper dreht.

3. Abstandhalter nach Anspruch 1-2, wobei die Spindel eine Innengewindefläche aufweist und die Welle eine Außengewindefläche aufweist, die für den Gewindeeingriff miteinander konfiguriert sind.

4. Abstandhalter nach Anspruch 1-3, ferner aufweisend eine Verriegelung, die dafür konfiguriert ist, der Drehung der Spindel zu widerstehen.

5. Abstandhalter nach Anspruch 1-4, ferner aufweisend eine Verriegelung mit mindestens einem Zahn, und wobei die Spindel ferner eine Mehrzahl von Zähnen an der anderen Fläche aufweist, die dafür konfiguriert sind, mit dem mindestens einen Zahn an der Verriegelung in Eingriff zu gehen.

6. Abstandhalter nach Anspruch 1-5, wobei jeder des ersten und des zweiten Arms mindestens einen Stift aufweist und der Körper entsprechende Öffnungen aufweist, die dafür konfiguriert sind, den ersten und den zweiten Arm mit dem Körper an den Stiften derart zu verbinden, dass die Arme in Bezug auf den Körper bewegbar sind.

7. Abstandhalter nach Anspruch 1-6, wobei die Öffnungen und die Stifte derart konfiguriert sind, dass der erste und der zweite Arm zu einer Drehung in Bezug auf den Körper fähig sind.

8. Abstandhalter nach Anspruch 1-6, wobei die Öffnungen und die Stifte derart konfiguriert sind, dass der erste und der zweite Arm zu einer Drehung und einer Verschiebung in Bezug auf den Körper fähig sind.

9. Abstandhalter nach Anspruch 1-8, wobei die Öffnungen und die Stifte derart konfiguriert sind, dass der erste und der zweite Arm zu einer Drehung, gefolgt von einer Verschiebung, in Bezug auf den Körper fähig sind.

10. Abstandhalter nach Anspruch 1-9, wobei sich der erste und der zweite Arm um ungefähr 90 Grad in mindestens eine Einsatzkonfiguration drehen, in welcher die Arme ungefähr quer zu der Längsachse des Körpers sind; wobei die Drehung aus einer Nicht-Einsatzkonfiguration erfolgt, in welcher die Arme im Wesentlichen parallel zu der Längsachse des Körpers sind.

11. Abstandhalter nach Anspruch 1-10, wobei sich der erste und der zweite Arm um ungefähr 90 Grad in mindestens eine Einsatzkonfiguration drehen, in welcher die Arme ungefähr quer zu der Längsachse des Körpers sind;
wobei die Drehung aus einer Nicht-Einsatzkonfiguration erfolgt, in welcher die Arme im Wesentlichen parallel zu der Längsachse des Körpers sind; und
wobei sich die Arme, auf die Drehung folgend, bei einer weiteren Betätigung in Bezug auf den Körper in einer zu der Längsachse des Körpers im Wesentlichen quer verlaufenden Richtung verschieben.

12. Abstandhalter nach Anspruch 1-11, wobei der Körper zu einer derartigen Befestigung an einem Abstandhalter-Einführinstrument konfiguriert ist, dass die Längsachse des Abstandhalter-Einführinstruments im Wesentlichen mit der Längsachse des Abstandhalters ausgerichtet ist.

13. Abstandhalter nach Anspruch 1-12, wobei die Spindel Ausnehmungen zum Aufnehmen eines Werkzeugs zum Drehen der Spindel in Bezug auf den Körper aufweist.

14. Abstandhalter nach Anspruch 1-13,wobei eine Drehung der Spindel in einer Richtung das Betätigungselement distal vorwärts bewegt, um mit den Nockenflächen in Eingriff zu gehen und die Arme in mindestens eine Einsatzkonfiguration zu versetzen, und eine Drehung der Spindel in einer entgegengesetzten Richtung das Betätigungselement proximal bewegt, um es den Armen zu gestatten, sich in mindestens eine Nicht-Einsatz- oder Zwischenkonfiguration einzuklappen.

15. Abstandhalter nach Anspruch 1-14, wobei jeder Arm ein Paar von Erweiterungen und einen Sattel aufweist, die eine im Wesentlichen U-förmige Konfiguration zum Lagern eines Dornfortsatzes definieren.

16. Abstandhalter nach Anspruch 1-14, wobei jeder Arm ein Paar von Erweiterungen aufweist, und wobei die Erweiterungen des ersten Arms derart voneinander beabstandet sind, dass sie den ersten Dornfortsatz aufnehmen und lagern können, und die Erweiterungen des zweiten Arms derart voneinander beabstandet sind, dass sie den zweiten Dornfortsatz aufnehmen und lagern können.

## Revendications

1. Espaceur implantable (10) pour un placement entre des apophyses épineuses, comprenant :
un corps (12) définissant un axe longitudinal ;
un premier bras (14) et un second bras (16) reliés au corps et capables de mouvement par rapport au corps de telle sorte que les premier et second bras peuvent tourner à l'opposé l'un de l'autre et à l'opposé de l'axe longitudinal du corps ;
chaque bras définissant une configuration pour recevoir une apophyse épineuse ;
chaque bras ayant une surface de came proximale (41, 43) ;
un ensemble actionneur (18) relié au corps ;
l'ensemble actionneur comprenant :
un actionneur (48) ayant au moins une surface de palier (58) ;
un arbre fileté (50) relié à l'actionneur et configuré pour se déplacer par rapport au corps ; et
une tige (86) ;
**caractérisé par le fait que**
l'ensemble actionneur est configuré pour se déplacer par rapport au corps de telle sorte qu'une rotation de la tige déplace l'actionneur de telle sorte que l'au moins une surface de palier entre en contact avec au moins l'une des surfaces de came pour déplacer les deux bras d'une configuration non déployée à une configuration déployée dans laquelle le premier bras reçoit et place une première apophyse épineuse et le second bras reçoit et place une seconde apophyse épineuse adjacente.

2. Espaceur selon la revendication 1, comprenant en outre un organe de retenue configuré pour retenir l'actionneur et la tige par rapport au corps de telle sorte que l'actionneur se déplace et la tige tourne par rapport au corps.

3. Espaceur selon les revendications 1 et 2, dans lequel la tige comprend une surface taraudée et l'arbre comprend une surface à filetage externe configurées pour être en prise par filetage l'une avec l'autre.

4. Espaceur selon les revendications 1 à 3, comprenant en outre un verrou configuré pour résister à une rotation de la tige.

5. Espaceur selon les revendications 1 à 4, comprenant en outre un verrou ayant au moins une dent et la tige comprenant en outre une pluralité de dents sur l'autre surface, configurées pour être en prise avec l'au moins une dent sur le verrou.

6. Espaceur selon les revendications 1 à 5, dans lequel chacun parmi les premier et second bras comprend au moins une broche et le corps comprend des ouvertures correspondantes configurées pour relier les premier et second bras au corps au niveau des broches de telle sorte que les bras sont déplaçables par rapport au corps.

7. Espaceur selon les revendications 1 à 6, dans lequel les ouvertures et les broches sont configurées de telle sorte que les premier et second bras sont capables d'une rotation par rapport au corps.

8. Espaceur selon les revendications 1 à 6, dans lequel les ouvertures et les broches sont configurées de telle sorte que les premier et second bras sont capables d'une rotation et d'une translation par rapport au corps.

9. Espaceur selon les revendications 1 à 8, dans lequel les ouvertures et les broches sont configurées de telle sorte que les premier et second bras sont capables d'une rotation, suivie d'une translation par rapport au corps.

10. Espaceur selon les revendications 1 à 9, dans lequel les premier et second bras tournent d'approximativement 90 degrés dans au moins une configuration déployée dans laquelle les bras sont approximativement transversaux à l'axe longitudinal du corps ; ladite rotation étant à partir d'une configuration non déployée dans laquelle les bras sont sensiblement parallèles à l'axe longitudinal du corps.

11. Espaceur selon les revendications 1 à 10, dans lequel les premier et second bras tournent d'approximativement 90 degrés dans au moins une configuration déployée dans laquelle les bras sont approximativement transversaux à l'axe longitudinal du corps ;
ladite rotation étant réalisée à partir d'une configuration non déployée dans laquelle les bras sont sensiblement parallèles à l'axe longitudinal du corps ; et
dans lequel à la suite de ladite rotation, lors d'un autre actionnement, les bras se déplacent en translation par rapport au corps dans une direction sensiblement transversale à l'axe longitudinal du corps.

12. Espaceur selon les revendications 1 à 11, dans lequel le corps est configuré pour une fixation à un instrument de pose d'espaceur de telle sorte que l'axe longitudinal de l'instrument de pose d'espaceur est sensiblement aligné avec l'axe longitudinal de l'espaceur.

13. Espaceur selon les revendications 1 à 12, dans lequel la tige comprend des encoches pour recevoir un outil pour une rotation sur la tige par rapport au corps.

14. Espaceur selon les revendications 1 à 13, dans lequel une rotation de la tige dans une première direction avance l'actionneur de manière distale pour mettre en prise les surfaces de came et déployer les bras dans au moins une configuration déployée, et une rotation de la tige dans une direction opposée déplace l'actionneur de manière proximale pour permettre aux bras de se replier dans au moins une configuration intermédiaire ou non déployée.

15. Espaceur selon les revendications 1 à 14, dans lequel chaque bras a une paire d'extensions et une selle définissant une configuration qui est sensiblement en forme de U pour placer une apophyse épineuse.

16. Espaceur selon les revendications 1 à 14, dans lequel chaque bras a une paire d'extensions, et les extensions du premier bras sont espacées l'une de l'autre de telle sorte qu'elles sont aptes à recevoir et placer la première apophyse épineuse et les extensions du second bras sont espacées l'une de l'autre de telle sorte qu'elles sont aptes à recevoir et placer la seconde apophyse épineuse.
